# EUROPÄISCHE PATENTSCHRIFT

(11) **EP 1 015 885 B1**
(45) Veröffentlichungstag und Bekanntmachung des Hinweises auf die Patenterteilung: **08.03.2006**
(21) Anmeldenummer: 98952583.7
(22) Anmeldetag: 31.08.1998
(51) Int. Cl.: C07K 1/22, G01N 33/53

(54) **AUFREINIGUNG VON SUBSTANZEN AUS EINER BIOLOGISCHEN PROBE**
PURIFICATION OF SUBSTANCES FROM A BIOLOGICAL SAMPLE
EPURATION DE SUBSTANCES PROVENANT D'UN ECHANTILLON BIOLOGIQUE

(30) Priorität: 08.09.1997 DE 19739218
(43) Veröffentlichungstag der Anmeldung: 05.07.2000
(73) Patentinhaber: Roche Diagnostics GmbH, 68305 Mannheim (DE)
(72) Erfinder: HÖSEL, Wolfgang, D-82327 Tutzing (DE); LENZ, Helmut, D-82327 Tutzing (DE); PETER, Jochen, D-83670 Bad Heilbrunn (DE)
(74) Vertreter: Weiss, Wolfgang
(86) Internationale Anmeldenummer: PCT/EP1998/005521
(87) Internationale Veröffentlichungsnummer: WO 1999/013330

(56) Entgegenhaltungen:
- EP-A- 0 378 197
- EP-A- 0 506 032
- WO-A-92/02818
- CROWE J. ET AL: 'One-step purification of recombinant proteins with the 6xHis tag and Ni-NTA resin' MOLECULAR BIOTECHNOLOGY Bd. 4, 1995, Seiten 247 - 258
- VOSS S.; SKERRA A.: 'Mutagenesis of a flexible loop in streptavidin leads to higher affinity for the Strep-tag II peptide and improved performance in recombinant protein purification' PROTEIN ENGINEERING Bd. 10, Nr. 8, 1997, Seiten 975 - 982
- YARMUSH M.L. ET AL: 'Immunoaffinity purification: basic principles and operational considerations' BIOTECH.ADV. Bd. 10, 1992, Seiten 413 - 446
- TSIOTIS G. ET AL: 'Isolation and structural characterization of trimeric cyanobacterial photosystem I complex with the help of recombinant antibody fragments' EUROPEAN JOURNAL OF BIOCHEMISTRY Bd. 231, 1995, Seiten 823 - 830
- KLEYMANN G. ET AL: 'Engineered Fv fragments as a tool for the one-step purification of integral multisubunit membrane protein complexes' BIO/TECHNOLOGY Bd. 13, Februar 1995, Seiten 155 - 160
- BRIZZARD B.L. ET AL: 'Immunoaffinity purification of FLAG epitope-tagged bacterial alkaline phosphatase using a novel monoclonal antibody and peptide elution' BIOTECHNIQUES Bd. 16, Nr. 4, 1994, Seiten 730 - 734, XP009026470

## Beschreibung

Die Erfindung betrifft ein Verfahren zur präparativen Aufreinigung einer Zielsubstanz aus einer biologischen Probe durch Immobilisierung der Zielsubstanz an einer Festphase über ein hochaffines Bindepaar und anschließende Elution durch Zugabe eines Partners des Bindepaares in freier Form. Weiterhin werden Reagenzienkits zur Durchführung des Verfahrens offenbart.

Die immunsorptive Reinigung von biologischen Substanzen, z.B. Makromolekülen wie etwa Proteinen, ist eine seit langem bekannte Methode, welche die effiziente Isolierung solcher Substanzen aus komplexen biologischen Materialien wie etwa Serum, Urin oder Zellaufschlüssen erlaubt (Eveleigh und Levy, J. Solid-phase Biochem. 2 (1977), 45-78; Cooper, Biochemische Arbeitsmethoden, pp. 222-241 (1981), Walter de Gruyter, Berlin; Pharmacia Fine Chemicals, Affinity Chromatography: Principles und Methods (1983), pp 92-95; Wawrazynczak und Cumber, in: "Immunochemical Protocols", M. M. Manson HRSG, Methods in Molecular Biology 10, Kapitel 32 (1992), Humana Press, Totowa, N.J. USA). Die Desorption der an die immobilisierten Antikörper gebundenen Substanzen erfolgt dabei üblicherweise mit unspezifischen Methoden, z.B. durch Zusatz von Puffern mit niedrigem (< 2,5) oder hohem (> 10) pH-Wert, chaotropen Reagenzien wie etwa Harnstoff oder Guanidinhydrochlorid, organischen Lösungsmitteln oder Detergenzien zum Elutionspuffer.

In einigen Fällen wurden auch spezifische Desorptionsmethoden beschrieben, z.B. zur Isolierung von Haptenen durch Verdrängung mit einem Haptenanalogon oder zur Isolierung von Proteinen durch Verdrängung mit einem niedermolekularen Peptid. Solche spezifischen Desorptionsvorgänge sind bei immunsorptiven Verfahren jedoch die Ausnahme, da sie nur mit einer sehr beschränkten Anzahl von Substanzen überhaupt durchführbar sind. Weitgehend werden daher die oben beschriebenen unspezifischen Desorptionsmethoden eingesetzt, vor allem die Elution bei niedrigem pH-Wert wie z.B. mit 1 M Propionsäure.

Bei immunsorptiven Reinigungsverfahren aus biologischen Materialien zeigt sich nun allerdings, daß die darin vorhandenen Biomoleküle, z.B. Proteine, zum Teil in beträchtlichem Ausmaß auch unspezifisch, d.h. nicht über eine Antikörper-Antigen-Wechselwirkung, an den Immunadsorber binden. Bei einer unspezifischen Desorption, z.B. mit saurem Puffer, werden diese Substanzen nun ebenfalls von dem Immunadsorber eluiert und liegen neben der gewünschten Substanz als Verunreinigung vor. Dies ist vor allem dann ein sehr großer Nachteil, wenn die aufzureinigende Substanz nur in sehr geringer Konzentration vorliegt, da sie dann nur einen geringen Anteil des insgesamt vom Immunadsorber eluierten Materials darstellt, so daß weitere aufwendige Reinigungsschritte notwendig sind, bevor die gewünschte Substanz in genügender Reinheit zur Verfügung steht, falls dies bei den zum Teil sehr geringen Mengen der Substanz überhaupt möglich ist.

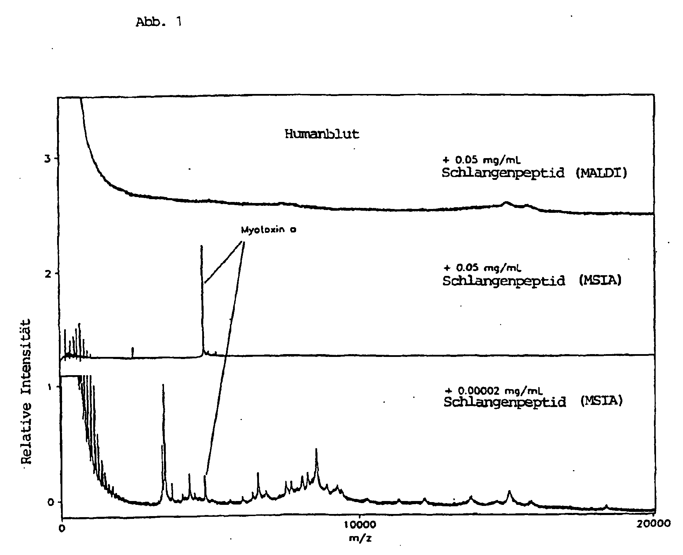

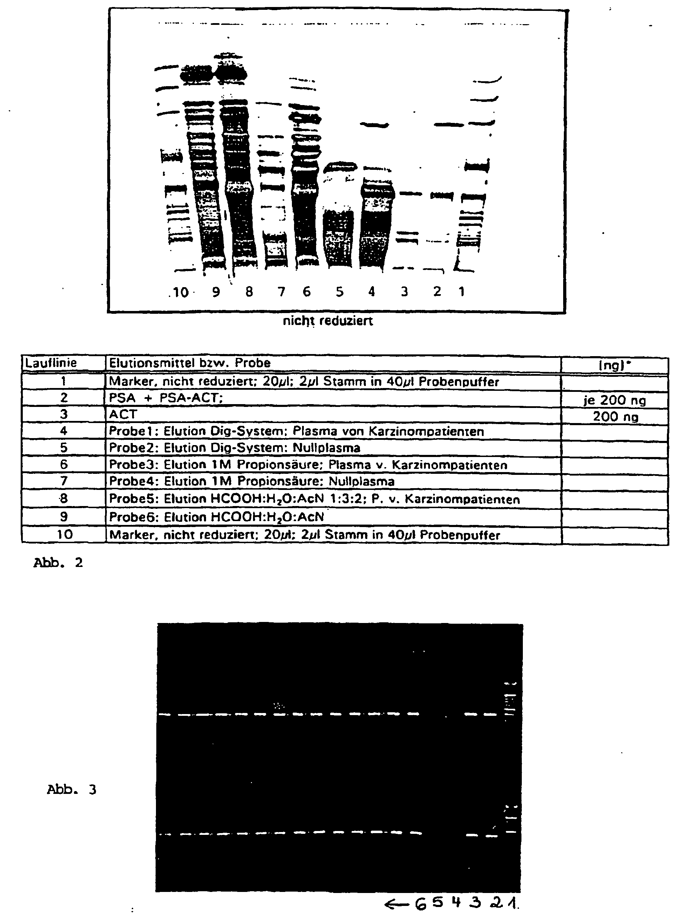

So liegt beispielsweise das Prostata-spezifische Antigen (PSA) in einer Konzentration von ca. 1 bis 1500 ng/ml in humanem Serum vor, während andere Proteine wie etwa humanes Serumalbumin (HSA) mit ca. 70 mg/ml in einer 10⁵ bis 10⁷-fach höheren Konzentration vorhanden sind. Da diese Proteine unspezifisch an den Immunadsorber binden, kommt es bei der immunsorptiven Reinigung einer in sehr geringen Konzentrationen vorliegenden Substanz wie z.B. PSA vor, daß bei einer unspezifischen Desorption die gewünschte Substanz nur einen sehr geringen Anteil des eluierten Gesamtproteins ausmacht und z.B. bei einer gelelektrophoretischen Auftrennung des Eluats überhaupt nicht identifiziert werden kann (vgl. z.B. Abb. 2). In diesem Fall ist eine Charakterisierung des Proteins, z.B. nach einem Proteaseverdau, aufgrund der Anwesenheit der verunreinigenden Proteine nicht oder nur sehr schwer möglich. Ähnliche Probleme treten auch bei anderen analytischen Methoden, wie etwa z.B. Massenspektrometrie auf, da auch hier eine Analyse aufgrund der Vielzahl von verunreinigenden Proteinen sehr schwierig oder unmöglich ist, vor allem wenn die genaue Masse des zu untersuchenden Proteins nicht bekannt ist. So ist zwar eine Kombination von MALDI-TOF Massenspektroskopie und lmmunsorption beschrieben, bei der das immunsorptiv gebundene Material durch die saure MALDI Matrix (Zimtsäurederivate gelöst in Trifluoressigsäure/Acetonitril) eluiert und direkt vermessen wird (Nelson et al., Anal. Chem. 67 (1995), 1 153-1158). Es ist aber zu erkennen, daß bei einer nur in sehr geringen Konzentrationen vorkommenden aufzureinigenden Substanz eine Vielzahl weiterer Proteine auftaucht, die teilweise bedeutend höhere Signale geben als die gewünschte Zielsubstanz (vgl. Abb. 1; entnommen aus Krone et al. Mass Spectrometric lmmunoassay, Poster Session ABRF'96: Biomolecular Techniques (1996), San Francisco, USA).

Kleymann et al., Bio/Technology, 1995, 13:155-160, beschreibt ein Verfahren zur Aufreinigung von Membranproteinen oder Membranproteinkomplexen unter Verwendung von speziell entwickelten Fv-Antikörperfragmenten, die gegen die aufzureinigenden Membranproteine oder Membranproteinkomplexe gerichtet sind und ein an den C-Terminus oder V_{H}-Kette durch gentechnische Verfahren fusioniertes Strep-Tag-Affinitätspeptid aufweisen, welches eine Adsorption an Streptavidin-gekoppeltes Chromatographiematerial und eine spezifische Desorption mittels Diaminobiotin ermöglicht.

Tsiotis et al., Eur. J. Biochem., 1995, 231:823-830, beschreiben die Aufreinigung des trimeren cyanobakteriellen Photosystem I-Komplexes unter Verwendung einer Strep-Tag-Affinitätspeptid/Streptavidin-Affinitätschromatographie mit spezifischen gegen den trimeren cyanobakteriellen Photosystem I-Komplex gerichteten Fv-Pragmenten, die ein Strep-Tag-Affinitätspeptid zur Bindung an Streptavidin-gekoppeltes Chromatographiematerial enthalten.

Voss und Skerra, Protein Engineering, 1997, 10:975-982, beschreiben die Mutagenese eines flexiblen Loops in Streptavidin und die damit verbundene höhere Affinität des Streptavidins für das Step-Tag II-Affinitätspeptid. Ferner beschreiben Voss und Skerra die Verwendung des mutierten Streptavidins zur Aufreinigung von Proteinen mit Strep-Tag 11-Affinitätspeptid.

Brizzard et al., Biotechniques, 1994 16:730-734, offenbart die Reinigung von alkalischer Phosphatase mit Hilfe von FLAG, welches an einen substratgebundenen Anti-FLAG-Antikörper binden kann. FLAG ist eine Sequenz aus 8 Aminosäuren, die natürlicherweise in der Phosphatase nicht vorhanden ist und deshalb durch gentechnische Verfahren eingebracht wird. Die Elution der an das Substrat gebundenen FLAG-Phosphatase geschieht durch einen Überschuss an freiem FLAG-Peptid.

Aufgabe der vorliegenden Erfindung war es daher, eine generell anwendbare Methode zur Aufreinigung von Substanzen aus biologischen Proben zu finden, bei der die Nachteile des Standes der Technik mindestens teilweise beseitigt sind. Insbesondere soll das Verfahren die Vorteile der spezifischen Immunsorption ausnutzen, während die Nachteile der unspezifischen Desorption vermieden werden. Insbesondere soll das erfindungsgemäße Verfahren eine Elution in einem im Wesentlichen neutralen pH-Bereich ermöglichen, um eine Schädigung der Zielsubstanzen verbunden mit einem Verlust an Immunreaktivität oder/und biologischer Aktivität durch extreme pH-Werte zu vermeiden.

Diese Aufgabe wird gelöst durch ein Verfahren zur Aufreinigung einer Zielsubstanz aus einer Probe, umfassend die Schritte:
(a) Adsorbieren der Zielsubstanz auf einer Festphase, wobei die Bindung der Zielsubstanz an die Festphase die Wechselwirkung zwischen einem ersten und einem zweiten Partner eines hochaffinen Bindepaa-res umfaßt, und wobei der erste Partner des Bindepaares direkt oder indirekt an die Zielsubstanz gebunden ist und wobei der zweite Partner des Bindepaares an die Festphase gebunden ist,
(b) Abtrennen nicht adsorbierter Probenbestandteile von der Festphase,
(c) Inkontaktbringen der Festphase mit dem ersten Partner des Bindepaares oder einem Analogon davon in freier Form, wobei die Zielsubstanz von der Festphase desorbiert wird und
(d) Abtrennen der Zielsubstanz von der Festphase.

Eine erste bevorzugte Ausführungsform der Erfindung, ist ein Verfahren zur Aufreinigung einer Zielsubstanz aus einer Probe, umfassend die Schritte:
(a) Bereitstellen einer Festphase mit einem immobilisierten Reaktanden R₁, wobei R₁ ein Konjugat von mindestens einer mit der Zielsubstanz spezifisch bindefähigen. Gruppe mit mindestens einer Gruppe umfassend einen ersten Partner eines hochaffinen Bindepaares ist, und wobei die Bindung von R₁ an die Festphase über einen immobilisierten Reaktanden R₂ erfolgt, der eine Gruppe umfassend den zweiten Partner des hochaffinen Bindepaares enthält, und wobei die Bindung zwischen erstem und zweitem Partner des hochaffinen Bindepaares reversibel sein muss,
(b) Inkontaktbringen der Probe mit der Festphase, wobei die Zielsubstanz über eine Bindung mit R₁ auf der Festphase adsorbiert wird,
(c) Abtrennen nicht adsorbierter Probenbestandteile von der Festphase,
(d) Inkontaktbringen der Festphase mit dem ersten Partner des Bindepaares oder einem Analogon davon in freier Form, wobei die Zielsubstanz als Komplex mit R₁ von der Festphase desorbiert wird, und
(e) Abtrennen des Komplexes von der Festphase.

Bei dieser Ausführungsform des erfindungsgemäßen Verfahrens wird ein mit der aufzureinigenden Zielsubstanz spezifischer bindefähiger Reaktand R₁ verwendet, der mit einem ersten Partner eines hochaffinen Bindepaares gekoppelt ist, wobei der erste Partner eine Haptengruppe ist. Weiterhin wird ein spezifisch mit dem ersten Bindepartner bindefähiger zweiter Reaktand R₂ verwendet, der als zweiten Partner des Bindepaares einen Anti-Hapten-Antikörper enthält und gegenüber dem ersten Bindepartner eine solche Affinität aufweist, daß der Reaktand R₁ durch den ersten Bindepartner in freier Form oder durch ein Derivat davon effizient verdrängt werden kann. Durch Elution mit dem freien ersten Bindepartner kann der Komplex aus dem Reaktanden R₁ und nachzuweisender Substanz sehr spezifisch und frei von unspezifisch adsorbierten Verunreinigungen von der Festphase eluiert werden. Das Prinzip und Ergebnis dieser Reinigungsmethode sowie ein Vergleich mit einer unspezifischen sauren Elution ist in Abbildung 2 am Beispiel der Isolierung von PSA und einem PSA/ACT-Komplex aus Humanserum dargestellt.

Das erfindungsgemäße Reinigungsprinzip kann auch zur direkten Isolierung einer Substanz eingesetzt werden, wenn diese mit einem ersten Bindepartner versehen werden kann und dessen Anwesenheit bei der weiteren Analytik oder Verwendung dieser Substanz nicht stört. Entsprechende Beispiele sind für die Isolierung von doppelt markierter DNA sowie von Fusionsproteinen gezeigt.

Eine zweite bevorzugte Ausführungsform der Erfindung betrifft somit ein Verfahren zur Aufreinigung einer Zielsubstanz, an die ein erster Partner eines hochaffinen Bindepaares gebunden ist, aus einer Probe, umfassend die Schritte:
(a) Bereitstellen einer Festphase, die einen immobilisierten zweiten Partner eines hochaffinen Bindepaares enthält,
(b) Inkontaktbringen der Probe mit der Festphase, wobei die Zielsubstanz über eine Bindung zwischen dem ersten und dem zweiten Partner des Bindepaares auf der Festphase adsorbiert wird,
(c) Abtrennen nicht adsorbierter Probenbestandteile von der Festphase,
(d) Inkontaktbringen der Festphase mit dem ersten Partner des Bindepaares oder einem Analogon davon in freier Form, wobei die Zielsubstanz von der Festphase desorbiert wird, und
(e) Abtrennen der Zielsubstanz von der Festphase.

Ishikawa beschreibt (EP 0 303 229) einen quantitativen lmmunoassay, bei dem ein an ein Polystyrolpartikel immobilisierter Komplex aus Anti-Dinitrophenyl (DNP)-Antikörper/DNP-markierte-Anti-Analyt-Antikörper/ Analyt/Anti-Analyt-Antikörper-Enzymkonjugat durch Zusatz von Dinitrophenol freigesetzt wird. Der freigesetzte Immunkomplex wird an einem zweiten Polystyrolpartikel, der einen gegen den haptenisierten Antikörper gerichteten Rezeptor enthält, gebunden und über die enzymatische Aktivität quantitativ bestimmt. Im Gegensatz zu dem bei Ishikawa beschriebenen Verfahren, ist die vorliegende Erfindung nicht auf die Entfernung eines im Meßmedium vorhanden markierten Reagenz, sondern auf die in einer biologischen Probe neben einer aufzureinigenden Substanz in sehr hohem Überschuß vorhandenen Begleitsubstanzen gerichtet. Es war somit vorher nicht abschätzbar, ob der von lshikawa beschriebene Desorptionsschritt überhaupt zur selektiven Entfernung dieser Begleitsubstanzen anwendbar ist. Dies gilt vor allem, weil bei dem erfindungsgemäßen Verfahren vorzugsweise eine direkte Analyse der vom Immunadsorber abgelösten Substanz und nicht wie bei Ishikawa ein weiterer Reinigungsschritt umfassend eine spezifische Bindung an eine weitere Festphase erfolgt. Überraschenderweise wurde festgestellt, daß eine solche weitere Bindung für eine effiziente Reinigung nicht notwendig ist, sondern daß schon in der spezifisch von der ersten Festphase eluierten Lösung eine hervorragende Abtrennung von Begleitsubstanzen zu beobachten ist.

Das erfindungsgemäße Verfahren ist für alle Reinigungsprobleme anwendbar, bei denen Substanzen, insbesondere biologische Moleküle, die vorwiegend in sehr geringer Konzentration vorliegen, aus komplexen biologischen Proben wie etwa Körperflüssigkeiten, z.B. Vollblut, Plasma, Serum, Urin, Sputum etc., aus tierischen oder pflanzlichen Geweben, Bodenproben oder Zellextrakten oder aus molekularbiologischen Reaktionsgemischen aufgereinigt werden müssen. Die aufzureinigende Substanz ist im weitesten Sinne ein Biomolekül, d.h. ein in einer biologischen Probe vorkommender Bestandteil. Beispiele für solche Substanzen sind Zellen, Zellfraktionen, Zellorganellen, virale Partikel, Polypeptide, Peptide, Glykoproteine, Lipoproteine, Polysaccharide, Nukleinsäuren, Hormone, Metaboliten, Neutransmitter und Mediatoren. Bevorzugte Beispiele für aufzureinigende Substanzen sind Polypeptide und Nukleinsäuren wie etwa DNA oder RNA Moleküle.

Gemäß der ersten bevorzugten Ausführungsform der Erfindung wird eine Zielsubstanz aufgereinigt, die nicht mit einem ersten Partner eines hochaffinen Bindepaares gekoppelt sein muß. Diese Ausführungsform des erfindungsgemäßen Verfahren ist insbesondere zur Aufreinigung von nichtmodifizierten, natürlicherweise in biologischen Proben vorkommenden Zielsubstanzen geeignet. Gemäß der zweiten bevorzugten Ausführungsform des erfindungsgemäßen Verfahrens, wird eine Zielsubstanz, die bereits mit einem ersten Partner eines hochaffinen Bindepaares gekoppelt ist, aufgereinigt. Diese Ausführungsform des Verfahrens ist insbesondere zur Aufreinigung von mit entsprechenden Gruppen modifizierten Zielsubstanzen aus molekularbiologischen Reaktionsansätzen, z.B. Hapten-modifizierten Nukleinsäuren aus Amplifikationsansätzen, oder zur Aufreinigung von rekombinanten Polypepfiden geeignet.

Die im erfindungsgemäßen Verfahren verwendete Festphase kann beliebige aus dem Stand der Technik bekannte Trägermaterialien enthalten. Bevorzugt sind partikuläre Festphasen wie etwa magnetische Mikropartikel.

Der in der ersten Ausführungsform verwendete Reaktand R₁ enthält bevorzugt nur eine Gruppe des ersten Bindepartners. Die spezifisch bindefähige Gruppe kann - je nach Art der nachzuweisenden Substanz - ein gegen die Substanz gerichteter Antikörper, eine mit der nachzuweisenden Substanz komplementäre Nukleinsäure, ein Lectin oder ein spezifisch an die nachzuweisende Substanz bindender biologischer Rezeptor sein. Vorzugsweise ist die spezifisch bindefähige Gruppe ein Antikörper (wobei dieser Begriff auch Antikörperfragmente oder Antikörperderivate umfaßt) oder eine Nukleinsäure (wobei dieser Begriff auch Nukleinsäureanaloga wie etwa peptidische Nukleinsäuren umfaßt).

Ein wesentliches Merkmal des erfindungsgemäßen Verfahrens ist, daß eine Elution der an die Festphase gebundenen Zielsubstanz durch Inkontaktbringen der Festphase mit dem ersten Partner des Bindepaares oder einem Analogon davon in freier bzw. löslicher Form möglich ist. Vorzugsweise werden die Partner des Bindepaares so ausgewählt, daß diese Elution unter physiologischen pH-Bedingungen, z.B. bei einem pH Bereich von 5 bis 8 erfolgen kann. Weiterhin ist es bevorzugt, daß die Partner des Bindepaares so ausgewählt werden, daß die Affinitätskonstante zwischen dem ersten und dem zweiten Partner des Bindepaares im Bereich vom 10⁶ l/mol bis 10¹⁰ l/mol liegt, so daß eine hochaffine Bindung erfolgt. Um eine gute Verdrängung durch den ersten Bindepartner in freier Form zu ermöglichen, soll die Bindung zwischen ersten und zweiten Bindepartner eine ausreichende Reversibilität aufweisen.

Vorzugsweise verwendet man als Haptengruppe, die der erste Partner des Bindepaares ist, eine niedermolekulare Substanz mit einem Molekulargewicht bis vorzugsweise maximal 4 kD, besonders bevorzugt bis maximal 2 kD. Als zweiten Partner des Bindepaares verwendet man einen gegen die Haptengruppe gerichteten Antikörper. Beispiele für geeignete Haptene sind Moleküle, die in der Lage sind, in einem Organismus eine Immunantwort hervorzurufen, die zur Produktion von spezifischen Anti-Hapten-Antikörpern führt. Spezifische Beispiele für Haptene sind Steroide wie etwa Progesteron oder synthetische Derivate davon, Cardenolide wie etwa Digoxin oder Digoxigenin, Fluoreszenzfarbstoffe wie etwa Fluorescein oder Derivate davon, Hormone wie etwa Schilddrüsenhormone, z.B. T3 oder T4, Arzneimittel wie Barbiturate oder Theophyllin, Metallkomplexe, insbesondere elektrochemilumineszenzfähige Metallkomplexe wie etwa Ruthenium- oder Rhodium-(Bispyridyl)₃₋Komplexe oder andere Verbindungen wie etwa Dinitrophenol oder Diphenylhydantoin. Substanzen, gegen die bereits hochspezifische Antikörper existieren, wie Digoxigenin, Fluorescein, Dinitrophenol, Schilddrüsenhormone, Ruthenium- oder Rhodium-Komplexe, Theophyllin, Barbiturate oder Diphenylhydantoin sind besonders bevorzugt.

In noch einer weiteren bevorzugten Ausführungsform verwendet man als ersten Partner des Bindepaares ein Peptidepitop und als zweiten Partner des Bindepaares einen gegen das Peptidepitop gerichteten Antikörper.

Ein den ersten Partner des Bindepaares bildendes Peptidepitop kann einerseits analog wie ein Hapten auf bekannte Weise durch Kopplung in Form eines aktivierten Derivats, z.B. eines Aktivesters, an den Reaktanden R₁, oder an die Zielsequenz gebunden werden. Wenn der Reaktand R₁ oder die Zielsubstanz eine Nukleinsäure ist, kann der erste Bindepartner auch durch eine enzymatische Reaktion eingeführt werden, z.B. durch Verwendung markierter Primer, markierter Oligonukleotidbausteineoder/undAnfügenmarkierterNukleinsäurefragmente.

Die Elution der Zielsubstanz bzw. des Komplexes aus R₁ und der Zielsubstanz von der Festphase erfolgt durch Inkontaktbringen der Festphase mit dem ersten Partner des Bindepaares oder einem Analogon davon in freier, bzw. löslicher Form. Eine möglichst quantitative Elution kann erreicht werden, wenn man den freien ersten Partner des Bindepaares in einem molaren Überschuß gegenüber dem an R₁ gebundenen ersten Partner des Bindepaares verwendet. Dieser molare Überschuß liegt vorzugsweise im Bereich von mindestens 50-fach, besonders bevorzugt 100-500-fach.

Weiterhin kann eine Verbesserung der Elution von der Festphase erreicht werden, wenn man den freien ersten Partner des Bindepaares in Form eines Konjugats einsetzt, umfassend ein Trägermolekül und mehrere daran gekoppelte Moleküle des ersten Partners des Bindepaares. Beispiele für geeignete Trägermoleküle sind Polypeptide wie etwa Polylysin, Albumin, unspezifische Antikörper etc. oder Polysaccharide wie etwa Dextrine. An dieses Trägermolekül können mehrere Moleküle des ersten Partner des Bindepaares nach bekannten Methoden, z.B. unter Verwendung von aktivierten Derivaten des ersten Partners gekoppelt werden. Beispiele für solche Konjugate sind polyhaptenylierte Trägermoleküle wie etwa Polylysin-Digoxigenin, Rinderserumalbumin-Digoxigenin etc.

Noch eine weitere Möglichkeit, die Elution von der Festphase zu verbessern, besteht darin, als freien ersten Partner des Bindepaares ein Analogon des an R₁ gebundenen ersten Partners des Bindepaares zu verwenden, wobei dieses freie Analogon eine höhere Bindungsaffinität an den zweiten Partner des Bindepaares aufweist als der an R₁ oder die Zielsubstanz gebundene erste Partner des Bindepaares. So kann - wenn der erste Partner des Bindepaares ein Hapten ist - als Analogon ein modifiziertes Hapten verwendet werden, an das der zweite Partner des Bindepaares eine höhere Affinität als an das gebundene Hapten aufweist. So kann man als gebundene Haptengruppe Digoxigenin, als Bindepartner einen Anti-Digoxin-Antikörper und als freien Bindepartner Digoxin verwenden. Analoge Beispiele sind die Verwendung von Nitrophenol als gebundenem Hapten, einem Anti-Dinitrophenol-Antikörper und Dinitrophenol als freiem Hapten, die Verwendung von T3 als gebundenem Hapten, Anti-T4-Antikörper und freiem T4 oder die Verwendung von Rhodium-Bispyridyl₃-Komplex als gebundenem Hapten, einemAnti-Rhodium-(Bispyridyl)₃-Komplex-Antikörper und Rhodium-(Bispyridyl)₃-Komplex als freiem Hapten.

Eine Verbesserung der Elution erhält man auch, wenn zwar als freier und gebundener Bindepartner die gleiche Gruppe verwendet wird, aber jeweils mit einem verschiedenen Linker. So kann man als gebundenen Partner eine Gruppe Hapten-Linker 1, einen gegen Hapten-Linker 2 gerichteten Antikörper und als freien Bindepartner die Gruppe Hapten-Linker 2 verwenden. Bei Peptidepitopen kann man als gebundenen Partner eine erste Peptidsequenz verwenden, einen gegen eine zweite, sich von der ersten Sequenz um eine Aminosäure unterscheidende Peptidsequenz gerichteten Antikörper und die zweite Peptidsequenz als freien Bindepartner verwenden.

Nach der Elution von der Festphase kann die Zielsubstanz einer Analyse, z.B. durch HPLC, Gelelektrophorese oder Massenspektroskopie unterzogen werden. Gegebenenfalls können noch weitere Reinigungsschritte durchgeführt, werden, z.B. eine Freisetzung der aufzureinigenden Substanz aus dem Komplex mit dem Reaktanden R₁. Außerdem können unerwünschte Anteile der aufzureinigenden Substanz abgespalten werden. So können beispielsweise bei Fusionsproteinen unerwünschte Anteile durch proteolytische Spaltung etwa mit Enterokinase, Faktor Xa oder IgA-Protease abgetrennt werden.

Eine spezifische Anwendung des erfindungsgemäßen Verfahrens ist die Aufreinigung rekombinanter Peptide oder Polypeptide, z.B. eukaryontischer Peptide oder Polypeptide, die in Bakterienzellen, z.B. in E.coli Zellen hergestellt werden. Dabei kann man Fusionspolypeptide herstellen, die eine erste Domäne, die spezifisch mit dem Reaktanden R₁ bindefähig ist, und eine zweite gewünschte Zieldomäne enthalten. Zwischen erster und zweiter Domäne kann eine Proteasespaltstelle eingeführt werden, so daß das Fusionspolypeptid nach der Desorption proteolytisch, z.B. mit einer immobilisierten Protease gespalten werden kann. Der im Reaktionsgemisch verbleibende Reaktand R₁ mit der daran gebundenen ersten Domäne des Fusionspolypeptids kann in einem weiteren Schritt durch Bindung an einen immobilisierten Reaktanden abgetrennt werden, so daß die gewünschte Zieldomäne in reiner Form erhalten wird.

Für den Fall, daß die Zielsubstanz, z.B. eine Peptid- oder Polypeptid-Zieldomäne ohne Abspaltung des zur Isolierung dienenden Komplexes R₁ und der ersten Domäne untersucht werden soll, kann an R₁ eine weitere, strukturell zum ersten Partner des Bindepaares unterschiedlichen Nachweisgruppe eingeführt werden. Mit Hilfe dieser weiteren Gruppe, kann eine Bindung an eine Festphase erfolgen, z.B. über das System Biotin/Streptavidin oder Hapten/Anti-Hapten-Antikörper, oder ein Konjugat zur Detektion, z.B. Biotin/Streptavidin-Enzym (beispielsweise Peroxidase oder alkalische Phosphatase), Hapten/Anti-Hapten-Antikörper-Enzym, angeheftet werden. Alternativ kann als Nachweisgruppe auch eine direkt nachweisbare Markierungsgruppe verwendet werden, z.B. ein lumineszierender Metallkomplex oder ein Fluoreszenzfarbstoff.

Noch ein weiterer Gegenstand der Erfindung ist ein Reagenzienkit zur Aufreinigung einer Zielsubstanz aus einer Probe umfassend:
(a) eine Festphase,
(b) einen Reaktanden R₁, wobei R₁ ein Konjugat von mindestens einer mit der aufzureinigenden Substanz spezifisch bindefähigen Gruppe mit mindestens einer Gruppe umfassend einen ersten Partner eines hochaffinen Bindepaares ist,
(c) einen Reaktanden R₂, der eine Gruppe umfassend den zweiten Partner des hochaffinen Bindepaares enthält, wobei R₂ an der Festphase gebunden ist oder eine an die Festphase bindefähige Gruppe enthält, und wobei die Bindung zwischen erstem und zweitem Partner des hochaffinen Bindepaares reversibel sein muss, und
(d) den ersten Partner des Bindepaares oder ein Analogon davon in freier Form,

wobei der erste Partner des hochaffinen Bindepaares eine Haptengruppe und der zweite Partner des hochaffinen Bindepaares ein gegen die Haptengruppe gerichteter Antikörper ist.

Zur Einführung des ersten Bindepartners in eine Zielsubstanz und die anschließende Aufreinigung der Zielsubstanz wird ein weiterer Reagenzienkit bereitgestellt umfassend:
(a) eine Festphase,
(bi) einen ersten Partner eines hochaffinen Bindepaares in einer Form, die zur Einführung in eine Zielsubstanz geeignet ist,
(bii) Mittel zur Einführung des ersten Partners des Bindepaares in die Zielsubstanz,
(c) den zweiten Partner des Bindepaares gebunden an der Festphase, und
(d) den ersten Partner des Bindepaares oder ein Analogon davon in freier Form.

Bevorzugte Ausführungsformen hinsichtlich der Bestandteile des Reagenzienkits sind wie vorstehend erläutert. Die Komponenten (a), (b) und (c) können als einziges Reagenz oder als mehrere separate Reagenzien vorliegen. Komponente (d) liegt separat von den anderen Komponenten vor. Die Komponenten des Reagenzienkits können in jeder beliebigen Form vorliegen, z.B. als abgepackte Einheiten, Lyophilisate, Lösungen, Suspensionen etc. Der Reagenzienkit wird vorzugsweise in einem Verfahren wie vorstehend beschrieben, eingesetzt.

Weiterhin wird die Erfindung durch die nachfolgenden Beispiele und Abbildungen erläutert:
- Abb. 1:: (Obere Spure) Normales MALDI Massenspektrum von humanem Blut, dem 0,05 mg/ml eines Schlangenpeptids (Myotoxin a) zugesetzt wurden. (Mittlere und untere Spuren) MALDI Spektren nach Bindung des Schlangenpeptids an einen Anti-Myotoxin a-Antikörper und Elution mit der sauren MALDI Matrix. Bei der unteren Spur, bei der nur 0,00002 mg/ml Myotoxin a zugesetzt worden war, sind deutlich die zahlreichen unspezifisch gebundenen und mit der sauren Matrix eluierten Peptide und Proteine aus dem Blut zu erkennen, die z.T. bedeutend höhere Signale als das Myotoxin a ergeben.
- Abb. 2:: Vergleich der gelelektrophoretischen Auftrennungen von aus Prostata Carcinom Plasma immunsorbiertem PSA und PSA/ACT Komplex nach Ablösen mit verschiedenen Elutionsmitteln. Eine Plasmaprobe ohne PSA und PSA/ACT wurde jeweils als Kontrolle mitgeführt.
- Abb. 3:: Ergebnis einer Agarosegelelektrophorese von DIG-markierten PCR Produkten nach Ablösen mit Digoxigenin-Lysin

### Experimentelle Beispiele

### Beispiel 1 Immunsorptive Isolierung von PSA aus Humanserum: Vergleich der Elution mit einem Hapten und mit Säuren

Die lmmunsorptionen erfolgen jeweils an Streptavidin-beschichteten Magnetbeads. Dazu wird jeweils 1 ml einer Bead-Suspension (0,72 mg/ml) pro Probe eingesetzt. Die Beads werden vor Zugabe der Proben dreimal mit jeweils 1 ml Waschlösung (Phosphat-gepufferte Salzlösung PBS, pH 7,2) gewaschen und der Überstand nach Separierung der Beads mit einem Magneten entfernt. Es werden sechs Ansätze der folgenden Zusammensetzung jeweils zu einem Aliquot von Beads zugegeben:

| | |
|---|---|
| Ansätze 1 und 2: | (1) 500 *µ*l monoklonaler Antikörper (MAK) Anti-Digoxigenin-IgG, biotinyliert (50 *µ*g/ml) und (2) 500 *µ*l MAK Anti-PSA-IgG, digoxigenyliert 50 *µ*g/ml) |
| Ansätze 3 bis 6: | 500 *µ*l MAK Anti-PSA-Fab', biotinyliert (16,6 *µ*g/ml) |

Nach Zugabe jeder Lösung erfolgt eine Inkubation für 60 min bei Raumtemperatur und unter Schütteln. Die Zugabe der Lösungen (1) und (2) zu den Ansätzen 1 und 2 erfolgt jeweils sequentiell. Danach werden die Beads vom Überstand abgetrennt, jeweils viermal mit Waschlösungen gewaschen und der Überstand entfernt.

Dann wird die Probe (Plasma) in einer Menge von 0,5 ml zu den einzelnen Ansätzen gegeben. Als Kontrolle wird Plasma ohne PSA verwendet. Nach Inkubation über 60 min und anschließendem viermaligen Waschen wie vorstehend beschrieben, werden die Überstände entfernt und folgende Elutionslösungen zugesetzt:

| | |
|---|---|
| Ansätze 1 und 2: | 200 *µ*l gesättigte Digoxigenin-Lysin-HCl Lösung, pH=7,2 |
| Ansätze 3 und 4: | 200 *µ*l Propionsäure 1 M |
| Ansätze 5 und 6: | 200 *µ*l Ameisensäure/Wasser/Acetonitril im Verhältnis 1:3:2 |

Die Ansätze werden 60 min bei Raumtemperatur geschüttelt und die Überstände nach Abtrennung der Beads in einer Speed Vac lyophilisiert. Die Lyophilisate werden dann in 10 *µ*l Wasser aufgenommen und für eine SDS-Polyacrylamid-Gelelektrophorese unter nichtreduzierenden Bedingungen eingesetzt, deren Ergebnis in Abb. 3 dargestellt ist.

### Ergebnis der Gelelektrophorese:

In den Ansätzen 3 bis 6, bei denen jeweils sauer eluiert wurde, sind sehr viele Proteinbanden aus dem Humanserum zu erkennen, die von unspezifischer Bindung an die Magnetbeads herrühren und durch die sauren Elutionsmittel abgelöst wurden. Dabei ist weiter zu sehen, daß das Acetonitril-haltige Elutionsmittel, welches für die anschließende MALDI-TOF-MS günstig ist, noch bedeutend mehr Proteine unspezifisch ablöst als die 1 M Propionsäure. Im Gegensatz dazu sind bei dem Ablösen mit Digoxigenin-Lysin-HCl neben den gesuchten Banden PSA und PSA/ACT nur die IgG Banden des digoxigenylierten Anti-PSA MAKs zu erkennen, sowie eine geringe Menge an Serumalbumin, das aufgrund seiner hohen Konzentration im Serum auch bei dieser spezifischen Elution der Beads zu einem geringen Teil abgelöst wird. Aufgrund der Vielzahl der bei der sauren Elution erhaltenen Proteinbanden ist es bei diesen Gelen sehr schwer, die spezifischen, gesuchten Proteine zu identifizieren. Dies erschwert dann auch eine weitere Analytik solcher Banden, z.B. durch MALDI-TOF-MS nach einem Proteaseverdau beträchtlich.

### Beispiel 2 Verwendung von lumineszierenden Metallkomplexen als Haptene

Entsprechend der in Beispiel 1 beschriebenen Vorgehensweise wurde eine immunsorptive Isolierung von PSA aus humanem Serum durchgeführt. Anstelle von Digoxigenin wurden als Haptene Ruthenium(Bispyridyl)₃₋Komplex und Rhodium(Bispyridyl)₃-Komplex und entsprechende Anti-Hapten-Antikörper verwendet. Die Elution erfolgte durch Zugabe der freien Metallkomplexe.

Wie in Beispiel 1 wurde eine spezifische Elution gefunden.

### Beispiel 3 Isolierung eines Fusionsproteins

Es wird ein Fusionsprotein konstruiert bestehend aus einem Protein 1 oder Peptid 1, gegen das ein digoxigenylierter Antikörper verfügbar ist, sowie dem gewünschten Zielprotein 2. Nach rekombinanter Expression dieses Fusionsproteins, z.B. in E.coli, wird ein Zellaufschluß durchgeführt. Das Homogenat wird mit den oben beschriebenen magnetischen Streptavidin Beads, welche mit MAK Anti-Digoxigenin beladen sind, eine Stunde unter Rühren bei Raumtemperatur geschüttelt. Nach magnetischer Trennung befindet sich im Überstand der reine Komplex aus dem digoxigenyliertem Antikörper und dem Fusionsprotein. Befindet sich zwischen dem Teil 1 und 2 des Fusionsproteins eine Proteasespaltstelle, z.B. für Enterokinase, kann mit Hilfe dieses Enzyms eine Spaltung des Fusionsproteins durchgeführt werden. Nach Abtrennung des digoxigenylierten Antikörpers mit dem daran gebundenen Protein 1, z.B. mit Protein A-Sepharose, liegt das reine Zielprotein 2 in der Lösung vor.

Der digoxigenylierte Antikörper kann eine weitere Nachweisgruppe tragen, z.B. ein von Digoxigenin verschiedenes Hapten. Mittels dieser weiteren Nachweisgruppe kann durch Bindung an einen Anti-Hapten-Antikörper eine Immobilisierung oder Detektion erreicht werden.

### Beispiel 4 Spezifische Desorption von digoxigenylierter DNA

Die lmmunsorptionen erfolgen an mit polyklonalem Anti-Dig-Antikörper beschichteten Mikrotiterplatten (AD-MTP, Boehringer Mannheim Produkt Kat. Nr. 1 754 289). Als DNA wird ein 397 bp langes PCR-Fragment (0,1 pmol/*µ*l) des Chloramphenicol-Gens (CAT) verwendet; dabei ist Primer 1 (TAT CCG GCC TTT ATT CAC ATT CTT G) 5' Digoxigenin-, Primer 2 (CCA GCG GCA TCA GCA CCT T) 5' Biotin-markiert.

### A) Desorptionsnachweis mit PCR

Je 50 *µ*l 1:10000 in Puffer (100 mmol/l Natriumphosphat pH 7,5, 50 mmol/l NaCl, 0,5 mmol/l EDTA, 1% Casein, 0,1 % Tween 20 und 0,02% MIT) mit 0,1 mg/ml Herings-DNA verdünntes CAT-Fragment werden in 8 Doppelansätzen für 30 min bei Raumtemperatur unter Schütteln an AD-MTP gebunden. Es wird zehnmal mit 250 *µ*l Waschpuffer (PBS mit 0,1 % Tween) gewaschen.

Die Elution erfolgt mittels Aufgabe von 50 *µ*l fertigem PCR-Ansatz (PCR Mastermix mit je 0,4 pmol/*µ*l Primer 1 und 2) mit 50% bis 0,78% Gehalt an Dig-Lysin gesättigtem bidestilliertem Wasser (1:2 Verdünnungreihe) bei 37 ° C, 1 h unter Schütteln. Als Negativkontrolle wird Puffer ohne Dig-Lysin und als Positivkontrolle eine Lösung mit 50% Dig-Lysin Gehalt und 1 *µ*l 1:200 verdünntes CAT-Fragment verwendet.

Anschließend wird mit den Ansätzen folgendes PCR-Programm gefahren:

| | |
|---|---|
| Denaturieren: | 94°C, 2 min |
| 20x bzw. 25x: | 94°C, 10 sec; 68°C, 30 sec |
| Finale Extension: | 72°C, 7 min |

Je 10 *µ*l pro PCR-Ansatz werden mittels 1,5% Agarosegelelektrophorese analysiert. Wie Abb. 3 zeigt, findet sich in allen Dig-Lysin haltigen PCRansätzen nach 20 (obere Reihe) bzw. 25 (untere Reihe) Zyklen das erzeugte CAT-Fragment analog der Positivkontrolle (Spuren 2 und 3 von rechts). Die Negativkontrolle (Spuren 4 und 5 von rechts) bleibt erwartungsgemäß leer.

### B) Desorptionsnachweis mit ELISA

Je 100 *µ*l 1:1000 in DNA-Konjugat-Verdünnungspuffer (100 mmol/I Natriumphosphat pH 7,5, 50 mmol/l NaCl, 0,5 mmol/l EDTA, 1% Casein, 0,1% Tween 20 und 0,02% MIT (Methylisothiazolon) verdünntes CAT-Fragment (Genbank ACC.NO.X65321) werden in 7 Doppelansätzen sowie einer Leerprobe ohne CAT für 30 min bei Raumtemperatur unter Schütteln an AD-Mikrotiterplatten gebunden. Es wird fünfmal mit 250 *µ*l Waschpuffer gewaschen.

Eine Elution erfolgt mittels Aufgabe von 100 *µ*l PBS mit 10% bis 0,001 % Dig-Lysin gesättigtem bidestilliertem Wasser (1:10 Verdünnungreihe) sowie 100% Kontrolle ohne Dig-Lysin bei 37°C, 1 h unter Schütteln.

Anschließend wird wie oben gewaschen und 30 min mit 100 *µ*l Streptavidin-Peroxidase-Konjugat (200 mU/ml) in Verdünnungspuffer detektiert. Dann wird nochmals wie oben gewaschen und mit 100 *µ*l TMB-Substrat (Tetramethylbenzidin) entwickelt. Die Reaktion wird mit 50 *µ*l 1 M H₂SO₄ gestoppt und im ELISA-Reader bei 450 nm vermessen.

Die Auswertung der ELISA-Daten zeigt eine spezifische Desorption der Dig-markierten DNA.

## Patentansprüche

1. Verfahren zur Aufreinigung einer Zielsubstanz aus einer Probe, umfassend die Schritte:
(a) Bereitstellen einer Festphase mit einem immobilisierten Reaktanden R₁, wobei R₁ ein Konjugat von mindestens einer mit einer Zielsubstanz spezifisch bindefähigen Gruppe mit mindestens einer Gruppe umfassend einen ersten Partner eines hochaffinen Bindepaares ist, und wobei die Bindung von R₁ an die Festphase über einen immobilisierten Reaktanden R₂ erfolgt, der eine Gruppe umfassend den zweiten Partner des hochaffinen Bindepaares enthält, und wobei die Bindung zwischen erstem und zweitem Partner des hochaffinen Bindepaares reversibel sein muss,
(b) Inkontaktbringen der Probe mit der Festphase, wobei die Zielsubstanz über eine Bindung mit R₁ auf der Festphase adsorbiert wird,
(c) Abtrennen nicht adsorbierter Probenbestandteile von der Festphase,
(d) Inkontaktbringen der Festphase mit dem ersten Partner des hochaffinen Bindepaares oder einem Analogon davon in freier Form, wobei die Zielsubstanz als Komplex mit R₁ von der Festphase desorbiert wird, und
(e) Abtrennen des Komplexes von der Festphase,
wobei man als ersten Partner des hochaffinen Bindepaares eine Haptengruppe und als zweiten Partner des hochaffinen Bindepaares einen gegen die Haptengruppe gerichteten Antikörper verwendet.

2. Verfahren nach Anspruch 1,
**dadurch gekennzeichnet,**
**dass** die aufzureinigende Substanz ausgewählt wird aus Zellen, Zellfraktionen, Zellorganellen, viralen Partikeln, Polypeptiden, Peptiden, Glykoproteinen, Lipoproteinen, Polysacchariden, Nukleinsäuren, Hormonen, Metaboliten, Neurotransmittem und Mediatoren.

3. Verfahren nach Anspruch 1 oder 2,
**dadurch gekennzeichnet,**
**dass** man eine partikuläre Festphase verwendet.

4. Verfahren nach einem der vorhergehenden Ansprüche,
**dadurch gekennzeichnet,**
**dass** man die Desorption von der Festphase unter physiologischen Bedingungen durchführt.

5. Verfahren nach einem der vorhergehenden Ansprüche,
**dadurch gekennzeichnet,**
**dass** man für die Desorption den freien ersten Partner des hochaffinen Bindepaares in einem molaren Überschuss gegenüber dem gebundenen ersten Partner des hochaffinen Bindepaares verwendet.

6. Verfahren nach einem der vorhergehenden Ansprüche,
**dadurch gekennzeichnet,**
**dass** man für die Desorption als freien ersten Partner des hochaffinen Bindepaares ein Analogon des gebundenen ersten Partners des hochaffinen Bindepaares verwendet, wobei das freie Analogon eine höhere Bindungsaffinität an den zweiten Partner des hochaffinen Bindepaares aufweist als der gebundene erste Partner des hochaffinen Bindepaares.

7. Verfahren nach einem der vorhergehenden Ansprüche,
**dadurch gekennzeichnet,**
**dass** das Verfahren weiterhin das Freisetzen der aufzureinigenden Substanz aus dem Komplex mit dem Reaktanden R₁ umfasst.

8. Verfahren nach einem der vorhergehenden Ansprüche,
**dadurch gekennzeichnet,**
**dass** der Reaktand R₁ eine vom ersten Partner des hochaffinen Bindepaares verschiedene Nachweisgruppe trägt.

9. Verwendung eines Verfahrens nach einem der Ansprüche 1 bis 8 zur Aufreinigung rekombinant hergestellter Polypeptide.

10. Verwendung nach Anspruch 9.
**dadurch gekennzeichnet,**
**dass** man ein Fusionspolypeptid herstellt, umfassend eine erste Domäne, die spezifisch mit dem ersten Partner des hochaffinen Bindepaares bindefähig ist, und eine zweite gewünschte Zieldomäne.

11. Reagenzienkit zur Aufreinigung einer Zielsubstanz aus einer Probe, umfassend:
(a) eine Festphase,
(b) einen Reaktanden R₁, wobei R₁ ein Konjugat von mindestens einer mit der aufzureinigenden Substanz spezifisch bindefähigen Gruppe mit mindestens einer Gruppe umfassend einen ersten Partner eines hochaffinen Bindepaares ist,
(c) einen Reaktanden R₂, der eine Gruppe umfassend einen zweiten Partner des hochaffinen Bindepaares enthält, wobei R₂ an der Festphase gebunden ist oder eine an die Festphase bindefähige Gruppe enthält, und wobei die Bindung zwischen erstem und zweitem Partner des hochaffinen Bindepaares reversibel sein muss, und
(d) den ersten Partner des hochaffinen Bindepaares oder ein Analogon davon in freier Form,
wobei der erste Partner des hochaffinen Bindepaares eine Haptengruppe und der zweite Partner des hochaffinen Bindepaares ein gegen die Haptengruppe gerichteter Antikörper ist.

## Claims

1. Method for purifying a target substance from a sample comprising the steps:
(a) providing a solid phase with an immobilized reactant R₁ where R₁ is a conjugate of at least one group that can bind specifically to a target substance with at least one group comprising a first partner of a high affinity binding pair, and where the binding of R₁ to the solid phase occurs by means of an immobilized reactant R₂ which contains a group comprising the second partner of the high affinity binding pair and where the binding between the first and second partner of the high affinity binding pair must be reversible,
(b) contacting the sample with the solid phase resulting in an adsorption of the target substance onto the solid phase via a binding to R₁,
(c) separating non-adsorbed sample components from the solid phase
(d) contacting the solid phase with the first partner of the high affinity binding pair or with an analogue thereof in a free form resulting in a desorption of the target substance as a complex with R₁ from the solid phase and
(e) separating the complex from the solid phase
wherein a hapten group is used as the first partner of the high affinity binding pair and an antibody directed against the hapten group is used as the second partner of the high affinity binding pair.

2. Method as claimed in claim 1,
**characterized in that**
the substance to be purified is selected from cells, cell fractions, cell organelles, viral particles, polypeptides, peptides, glycoproteins, lipoproteins, polysaccharides, nucleic acids, hormones, metabolites, neurotransmitters and mediators.

3. Method as claimed in claim 1 or 2,
**characterized in that**
a particulate solid phase is used.

4. Method as claimed in one of the previous claims,
**characterized in that**
the desorption from the solid phase is carried out under physiological conditions.

5. Method as claimed in one of the previous claims,
**characterized in that**
for the desorption the free first partner of the high affinity binding pair is used in a molar excess relative to the bound first partner of the high affinity binding pair.

6. Method as claimed in one of the previous claims,
**characterized in that**
for the desorption an analogue of the bound first partner of the high affinity binding pair is used as the free first partner of the high affinity binding pair where the free analogue has a higher binding affinity for the second partner of the high affinity binding pair than that of the bound first partner of the high affinity binding pair.

7. Method as claimed in one of the previous claims,
**characterized in that**
the method additionally comprises the release of the substance to be purified from the complex with the reactant R₁.

8. Method as claimed in one of the previous claims,
**characterized in that**
the reactant R₁ carries a detection group that is different from the first partner of the high affinity binding pair.

9. Use of a method as claimed in one of the claims 1 to 8 to purify recombinantly produced polypeptides.

10. Use as claimed in claim 9,
**characterized in that**
a fusion polypeptide is produced comprising a first domain which can bind specifically to the first partner of the high affinity binding pair and a second desired target domain.

11. Reagent kit for the purification of a target substance from a sample comprising:
(a) a solid phase,
(b) a reactant R₁ where R₁ is a conjugate of at least one group that can specifically bind to the substance to be purified with at least one group comprising a first partner of a high affinity binding pair,
(c) a reactant R₂ which contains a group comprising the second partner of the high affinity binding pair where R₂ is bound to the solid phase or contains a group which can bind to the solid phase and where the binding between the first and second partner of the high affinity binding pair must be reversible, and
(d) the first partner of the high affinity binding pair or an analogue thereof in a free form
wherein a hapten group is used as the first partner of the high affinity binding pair and an antibody directed against the hapten group is used as the second partner of the high affinity binding pair.

## Revendications

1. Procédé de purification d'une substance cible d'un échantillon comprenant les étapes de
(a) mise à disposition d'une phase solide avec un réactif R₁ immobilisé, R₁ étant un conjugué d'au moins un groupe capable de se lier spécifiquement avec une substance cible avec au moins un groupe comprenant un premier partenaire d'une paire de liaisons à haute affinité, et la liaison de R₁ à la phase solide étant effectuée via un réactif immobilisé R₂, qui contient un groupe comprenant le deuxième partenaire de la paire de liaisons à haute affinité, et la liaison entre le premier et le deuxième partenaire de la paire de liaisons à haute affinité devant être réversible,
(b) mise en contact de l'échantillon avec la phase solide, la substance cible étant adsorbée sur la phase solide via une liaison avec R₁,
(c) séparation des constituants de l'échantillon non adsorbés de la phase solide,
(d) mise en contact de la phase solide avec le premier partenaire de la paire de liaisons à haute affinité ou d'un analogue de celui-ci sous forme libre, dans laquelle la substance cible est désorbée de la phase solide sous la forme de complexe avec R₁, et
(e) séparation du complexe de la phase solide,
dans lequel on utilise comme premier partenaire de la paire de liaisons à haute affinité un groupe haptène et comme deuxième partenaire de la paire de liaisons à haute affinité un anticorps dirigé contre le groupe haptène.

2. Procédé selon la revendication 1, **caractérisé en ce que** la substance à purifier est choisie parmi les cellules, les fractions de cellules, les organelles cellulaires, les particules virales, les polypeptides, les peptides, les glycoprotéines, les lipoprotéines, les polysaccharides, les acides nucléiques, les hormones, les métabolites, les neurotransmetteurs et les médiateurs.

3. Procédé selon la revendication 1 ou 2, **caractérisé en ce que** l'on utilise une phase solide particulaire.

4. Procédé selon l'une des revendications précédentes, **caractérisé en ce que** l'on effectue la désorption de la phase solide dans des conditions physiologiques.

5. Procédé selon l'une des revendications précédentes, **caractérisé en ce que** l'on utilise pour la désorption le premier partenaire libre de la paire de liaisons à haute affinité dans un excès molaire par rapport au premier partenaire lié de la paire de liaisons à haute affinité.

6. Procédé l'une des revendications précédentes, **caractérisé en ce que** l'on utilise pour la désorption comme premier partenaire libre de la paire de liaisons à haute affinité un analogue du premier partenaire lié de la paire de liaisons à haute affinité, l'analogue libre présentant une affinité de liaison pour le deuxième partenaire de la paire de liaisons à haute affinité supérieure à celle du premier partenaire lié de la paire de liaisons à haute affinité.

7. Procédé selon l'une des revendications précédentes, **caractérisé en ce que** le procédé comprend de plus la libération de la substance à purifier du complexe avec le réactif R₁.

8. Procédé selon l'une des revendications précédentes, **caractérisé en ce que** le réactif R₁ porte un groupe d'identification différent de la paire de liaisons à haute affinité.

9. Utilisation d'un procédé selon l'une des revendications 1 à 8 pour la purification de polypeptides préparés par recombinaison.

10. Procédé selon la revendication 9, **caractérisé en ce que** l'on prépare un polypeptide de fusion, comprenant un premier domaine, qui est capable de se lier spécifiquement avec le premier partenaire de la paire de liaisons à haute affinité, et un deuxième domaine cible souhaité.

11. Kit de réactifs pour la purification d'une substance cible d'un échantillon comprenant :
(a) une phase solide,
(b) un réactif R₁, dans lequel R₁ est un conjugué d'au moins un groupe capable de se lier spécifiquement avec la substance à purifier avec au moins un groupe comprenant un premier partenaire d'une paire de liaisons à haute affinité,
(c) un réactif R₂, qui contient un groupe comprenant un deuxième partenaire de la paire de liaisons à haute affinité, R₂ étant lié à la phase solide ou contient un groupe capable de se lier à la phase solide, et la liaison entre le premier et le second partenaire de la paire de liaisons à haute affinité devant être réversible, et
(d) le premier partenaire de la paire de liaisons à haute affinité ou un analogue de celui-ci sous forme libre,
dans lequel le premier partenaire de la paire de liaisons à haute affinité est un groupe haptène et le deuxième partenaire de la paire de liaisons à haute affinité est un anticorps dirigé contre le groupe haptène.
